# FASCICULE DE BREVET EUROPEEN

(11) **EP 2 152 661 B1**
(45) Date de publication et mention de la délivrance du brevet: **06.10.2010**
(21) Numéro de dépôt: 08805816.9
(22) Date de dépôt: 20.05.2008
(51) Int. Cl.: C07C 209/48, C07C 211/03, C07C 211/21, C07C 253/22, C07C 255/03, C07C 255/07, C07D 317/36, C07D 319/02

(54) **PROCEDE DE CO-PRODUCTION DE CARBONATES CYCLIQUES ET DE NITRILES ET/OU D'AMINES GRAS**
KOPRODUKTION VON CYCLISCHEN CARBONATEN UND VON NITRILEN UND/ODER FETTAMINEN
COPRODUCTION OF CYCLIC CARBONATES AND OF NITRILES AND/OR OF FATTY AMINES

(30) Priorité: 24.05.2007 FR 0755236
(43) Date de publication de la demande: 17.02.2010
(73) Titulaire: ARKEMA FRANCE, 92700 Colombes (FR)
(72) Inventeur: DUBOIS, Jean-Luc, F-69390 Millery (FR); GILLET, Jean-Philippe, F-69530 Brignais (FR)
(86) Numéro de dépôt international: PCT/FR2008/050870
(87) Numéro de publication internationale: WO 2008/145941

(56) Documents cités:
- EP-A- 0 625 519
- US-A- 4 801 730
- LI ET AL: "Synthesis of cyclic carbonates from urea and diols over metal oxides" CATALYSIS TODAY, ELSEVIER, vol. 115, no. 1-4, 30 juin 2006 (2006-06-30), pages 111-116, XP005451301 ISSN: 0920-5861
- BHANAGE B M ET AL: "TRANSESTERIFICATION OF UREA AND ETHYLENE GLYCOL TO ETHYLENE CARBONATE AS AN IMPORTANT STEP FOR UREA BASED DIMETHYL CARBONATE SYNTHESIS" GREEN CHEMISTRY, ROYAL SOCIETY OF CHEMISTRY, CAMBRIDGE,, GB, vol. 5, no. 4, 2003, pages 429-432, XP008056370 ISSN: 1463-9262

## Description

L'invention vise un procédé conjugué de synthèse d'une part de nitriles et/ou amines gras, et d'autre part de carbonates de polyols, à partir d'huiles naturelles.

L'évolution actuelle en matière d'environnement conduit dans les domaines de l'énergie et de la chimie à privilégier l'exploitation des matières premières naturelles provenant d'une source renouvelable. Ces contraintes environnementales imposent également d'éviter le transport et le stockage de matières premières dangereuses.

Un exemple de procédé industriel utilisant un acide gras comme matière première est celui de la fabrication de nitriles et/ou d'amines gras à partir d'un acide gras qui peut être issu d'un triglycéride animal ou végétal. Ce procédé est décrit dans l'encyclopédie Kirk-Othmer Vol 2, 4° Edition, page 411. L'amine grasse est obtenue en plusieurs étapes. La première consiste en une méthanolyse ou une hydrolyse d'une huile végétale ou d'une graisse animale produisant respectivement l'ester méthylique d'un acide gras ou un acide gras. L'ester méthylique de l'acide gras peut ensuite être hydrolysé pour former l'acide gras. Ensuite, l'acide gras est transformé en nitrile par réaction avec l'ammoniac, et finalement en amine par hydrogénation du nitrile ainsi obtenu.

La préparation des amines grasses à partir des acides gras via un nitrile date des années 1940. Le schéma réactionnel de la synthèse des nitriles peut se résumer de la façon suivante.

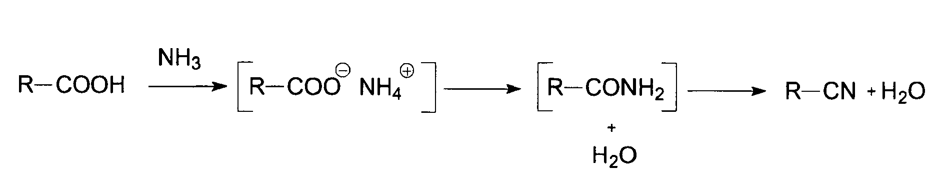

Il existe 2 types de procédés basés sur ce schéma réactionnel : un procédé batch en phase liquide qui est pratiqué par la société CECA et un procédé continu en phase vapeur.

Dans le procédé batch, on charge l'acide gras ou un mélange d'acides gras avec un catalyseur qui est généralement un oxyde métallique et le plus fréquemment l'oxyde de zinc. On porte le milieu réactionnel jusqu'à environ 150°C sous agitation, puis on commence à introduire l'ammoniac gazeux. Dans un premier temps, on forme un sel d'ammonium ou savon d'ammonium. La température du milieu réactionnel est ensuite portée aux alentours de 250°-300°C toujours sous introduction d'ammoniac. Le sel d'ammonium se transforme en amide avec libération d'une première molécule d'eau. Puis, dans un deuxième temps et avec l'aide du catalyseur, l'amide se transforme en nitrile avec formation d'une deuxième molécule d'eau. Cette eau formée est éliminée en continu du réacteur en entraînant l'ammoniac qui n'a pas réagi et un peu de chaînes grasses parmi les plus légères. Le passage par un déflegmateur rétrograde les composés gras vers le milieu réactionnel alors que les eaux ammoniacales sont envoyées vers un système de récupération de l'ammoniac qui peut être recyclé. La réaction est terminée quand il n'y a plus de fonction acide et que la teneur en amide est conforme aux spécifications.

Dans le procédé continu, la réaction a lieu en phase vapeur à des niveaux de température élevés et généralement sur un lit fixe d'alumine dopée ou non.

L'avantage du procédé batch est de conduire à des nitriles plus purs car ils sont généralement distillés, alors que ce n'est pas le cas en procédé continu. Par contre, le procédé en continu est plus avantageux lorsqu'il s'agit de nitriles à chaînes grasses insaturées ou poly-insaturées qui sont thermiquement plus sensibles que des composés à chaînes grasses saturées. En effet, dans ce procédé le temps de séjour est faible et l'indice d'iode, traduisant le nombre d'insaturations, est mieux conservé.

L'étape de synthèse des amines grasses consiste en une hydrogénation classique des nitriles gras. Les catalyseurs sont nombreux mais préférentiellement, on utilise le nickel de Raney ou le cobalt de Raney. Afin de favoriser la formation de l'amine primaire, on opère avec une pression partielle d'ammoniac, alors que dans le cas où l'on souhaiterait obtenir une amine secondaire on ne met pas de pression partielle d'ammoniac et on élimine si possible l'ammoniac formé en cours de réaction.

De nombreuses demandes de brevets décrivent les conditions pour effectuer la synthèse de nitriles d'acides gras ou d'amines grasses à partir de triglycérides ou d'esters méthyliques. On peut citer par exemple le document JP 2000-7637 qui décrit la synthèse de nitriles à partir d'esters méthyliques d'acides gras linéaires ou ramifiés insaturés ou non, comportant de 6 à 22 atomes de carbone, et d'ammoniac à une température allant de 180° à 350°C en présence d'un catalyseur de type oxyde de niobium. Le brevet US 6,005,134, décrit un procédé de synthèse de nitriles aliphatiques à partir d'acides carboxyliques en C₆-C₂₂, d'alkyl esters d'acides carboxyliques en C₆-C₂₂ ou à partir de triglycérides, la réaction avec l'ammoniac étant réalisée en présence d'un catalyseur comprenant au moins du titane. Les résultats obtenus indiquent cependant des rendements inférieurs en partant d'esters ou de triglycérides, comparativement aux acides. Dans le brevet JP 10-195035, une catalyse à l'oxyde de zirconium dopé au fer conduit à des rendements excellents en nitrile, mais les auteurs mentionnent la présence d'amines légères liées à la formation de méthanol dans le cas des esters méthyliques. Le brevet US 4,801,730 décrit un procédé pour préparer simultanément des nitriles d'acides gras et du glycérol à partir de triglycérides. La réaction est effectuée en 2 étapes, avec la production dans un premier temps de glycérol, d'eau, d'acides gras, d'amides d'acides gras et de nitriles par réaction avec l'ammoniac, puis en faisant à nouveau réagir le mélange d'acides gras, amides gras et nitriles gras avec de l'ammoniac pour convertir les acides et amides gras en nitriles.

Le procédé de synthèse des nitriles et/ou amines gras, opéré de manière industrielle à partir d'acides gras depuis plusieurs décennies donne globalement satisfaction. Cependant, il présente un certain nombre d'inconvénients. L'inconvénient principal est que sa mise en oeuvre est en pratique asservie à l'accès à une matière première spécifique, l'ammoniac en particulier, qui nécessite des précautions de stockage et d'utilisation coûteuses.

Lorsqu'un triglycéride est utilisé comme source d'acide gras pour le procédé de synthèse des nitriles et/ou amines gras, le procédé produit, lors de sa première étape de méthanolyse du triglycéride, du glycérol mais aussi du méthanol lors de l'étape consécutive d'hydrolyse. Ce méthanol est alors purifié et concentré pour être recyclé pour effectuer l'étape de méthanolyse initiale.

Il existe plusieurs voies de synthèse du carbonate de glycérol. Certains procédés utilisent le phosgène, d'autres une oxydation du glycérol en présence de CO, JP 6 157509 et US 5,359,094, d'autres encore une transestérification avec un autre carbonate, et finalement d'autres procédés utilisent une transestérification du glycérol avec l'urée.

Depuis de nombreuses années, le carbonate de glycérol est produit par transestérification avec du carbonate d'éthylène en catalyse homogène US 2,915,529, FR 2 733 232, US 5,091,543. D'autres procédés de synthèse de carbonate utilisent des catalyseurs hétérogènes US 4,691,041.

La demande de brevet EP 955 298 décrit un procédé de transestérification du glycérol par de l'urée, utilisant un catalyseur portant des sites acides de Lewis associé à un ou des contre ions anioniques d'hétéroatomes. Les expérimentations ont montré que le glycérol apporté pouvait être sous la forme de glycérine de qualité technique.

Le brevet US 6,495,703 décrit aussi un procédé de production de carbonate de glycérol par transestérification de l'urée. La réaction est effectuée de préférence en présence d'un agent déshydratant.

Il faut noter que les réactions de carbonatation utilisant de l'urée ont déjà été décrites pour d'autres polyols EP 443 758 et EP 581 131.

Dans le procédé de synthèse des nitriles et/ou amines gras, il est nécessaire d'importer et de stocker des quantités importantes d'ammoniac, ce qui représente outre le problème du transport, un risque majeur en cas de fuite, non seulement pour le personnel mais aussi pour le voisinage. Ce stockage impose par conséquent des contraintes importantes au site exploitant ce type de procédé.

Le problème à résoudre est de limiter ces risques environnementaux tout en maintenant les performances du procédé ou même mieux en les améliorant.

L'invention vise donc un procédé conjugué de production de nitriles et/ou amines gras et de carbonates de polyol à partir d'un triglycéride naturel, saturé ou insaturé, dans lequel le même triglycéride naturel servira de matière première pour la synthèse de nitriles et/ou amines gras d'une part, et de carbonates de polyol d'autre part. La synthèse des carbonates de polyol est réalisée par action de l'urée sur le polyol, l'ammoniac ainsi produit pourra être utilisé comme réactif, d'une part pour l'étape d'ammoniation de l'acide gras issu du triglycéride lors de la synthèse du nitrile, d'autre part pour l'étape d'hydrogénation du nitrile lors de la synthèse de l'amine primaire.

L'invention vise un procédé conjugué de production de nitriles et/ou amines gras et de carbonates de polyol à partir d'une huile naturelle comportant les zones suivantes :
I) zone de méthanolyse ou d'hydrolyse d'une huile naturelle contenant un triglycéride d'au moins un acide gras produisant, d'une part le méthanolate de l'acide gras ou l'acide gras répondant à la formule générale R-COOR₁, dans laquelle R est un radical alkyl saturé ou insaturé comportant de 7 à 21 atomes de carbone, de préférence de 11 à 17 atomes de carbone, R₁ est soit CH₃ dans le cas de la méthanolyse, soit H dans le cas de l'hydrolyse, et d'autre part du glycérol,
II) zone de synthèse d'une amine grasse comportant les étapes suivantes a) transformation optionnelle tout d'abord du méthanolate de l'acide gras en un acide gras par une hydrolyse de l'ester issu de l'étape I), suivie en b) d'une réaction d'ammoniation avec de l'ammoniac de l'acide ou du méthanolate de l'acide gras issu de la zone I ou de l'acide issu de l'étape a), pour former un nitrile, puis en c) d'une réduction par l'hydrogène du composé résultant de l'étape IIb, éventuellement en présence d'ammoniac, pour obtenir l'amine correspondante,
III) zone de synthèse d'un carbonate de polyol par réaction de l'urée, soit directement sur le glycérol, soit sur du propylène glycol ou de l'éthylène glycol obtenus suite à une réduction par hydrogénation du glycérol, la réaction du polyol avec l'urée produisant de l'ammoniac,
IV) zone de récupération de l'ammoniac issue de la zone III, ainsi que celle issue de la zone IIb où la réaction est réalisée avec un excès d'ammoniac, et éventuellement celle issue de la zone IIc, pour servir d'alimentation pour l'ammoniation de l'étape IIb et éventuellement l'hydrogénation de l'étape IIc de la zone II.

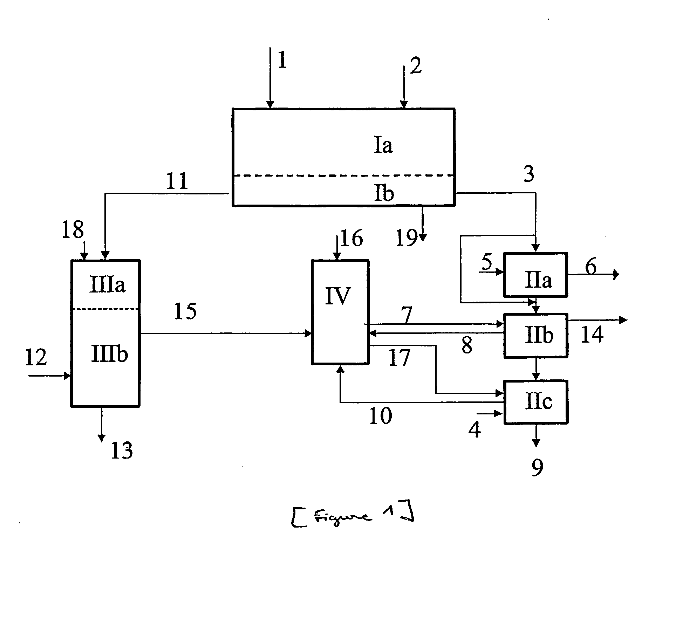

Le procédé de l'invention est décrit en référence au schéma simplifié annexé en figure 1.

La zone I est alimentée par la ligne 1 en huile naturelle contenant un triglycéride d'une part, et d'autre part en méthanol ou eau par la ligne 2. Une fois la réaction de méthanolyse ou d'hydrolyse du triglycéride réalisée dans la zone la selon la réaction suivante : 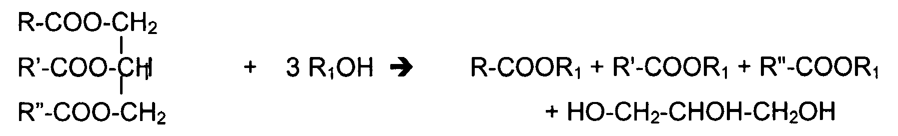 R, R', R" étant des radicaux alkyl saturés ou insaturés comportant de 7 à 21 atomes de carbone, de préférence de 11 à 17 atomes de carbone, R, R', R" identiques ou différents, et R₁ étant soit CH₃ soit H.

On sépare en général par décantation dans la zone lb, d'une part l'ester méthylique de l'acide gras ou l'acide gras du triglycéride qui est alors adressé par la ligne 3 à la zone II et, d'autre part le glycérol qui est adressé par la ligne 11 à la zone III. Les composés résiduels sont extraits de la zone 1 par la ligne 19.

L'ester méthylique de l'acide gras formé en zone la est ensuite transféré en zone IIa où il est soumis à une hydrolyse afin d'obtenir l'acide gras correspondant. L'eau est introduite par la ligne 5 et le méthanol extrait par la ligne 6. Ce méthanol, éventuellement purifié, pourra être recyclé en zone 1 pour la réaction de transestérification.

L'acide gras issu de la zone IIa ou issu directement de la zone Ib, ou le méthanolate de l'acide gras issu de la zone Ib est introduit dans la zone IIb où il est soumis à une réaction d'ammoniation avec de l'ammoniac en excès pour obtenir le nitrile gras selon le processus réactionnel suivant :

R-COOH + NH₃ → R-CN +2H₂O

ou

R-COOCH₃ + NH₃ → R-CN + H₂O + CH₃OH

L'ammoniac est introduit par la ligne 7. L'excès d'ammoniac et l'eau produite à partir de l'acide gras sont extraits par la ligne 8 et envoyés à la section IV pour purification. L'excès d'ammoniac et l'eau et le méthanol produits à partir du méthanolate de l'acide gras sont extraits par la ligne 14 et/ou 8, le méthanol produit pouvant être recyclé, après purification, en zone I pour la réaction de transestérification.

Le nitrile issu de la zone IIb est introduit dans la zone IIc où il est soumis à une réduction par l'hydrogène, éventuellement en présence d'ammoniac, selon le processus réactionnel suivant :

R-CN + 2 H₂ (NH₃) → R-CH₂-NH₂ ,

ou

2 R-CN + 4 H₂ → R-CH₂-NH-CH₂-R + NH₃

L'ammoniac est introduit dans la zone IIc par la ligne 17 et l'hydrogène par la ligne 4. L'amine grasse est extraite de la zone II par la ligne 9, et l'excès d'ammoniac est renvoyé à la section IV pour purification. En général, l'ammoniac est pour toutes ces opérations sous forme gazeuse, mais peut être sous forme liquéfiée, aqueuse ou anhydre suivant les flux.

Le glycérol issu de la zone I est adressé par la ligne 11 à la zone III dans laquelle on injecte par la ligne 12 de l'urée pour former le carbonate de glycérol selon le processus réactionnel suivant :

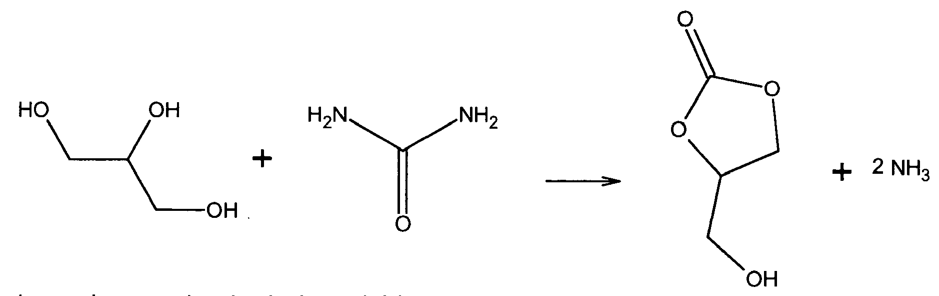

Le carbonate de glycérol synthétisé est extrait de la zone III par la ligne 13 et l'ammoniac formé est adressée à la zone IV par la ligne 15.

Dans le cas où l'on souhaite synthétiser un carbonate cyclique de propylène glycol 1,2 ou 1,3, la zone III est divisée en deux zones IIIa et IIIb ; dans la zone IIIa on procède à une réduction partielle du glycérol par injection d'hydrogène par la ligne 18 et le (ou les) propylène glycol(s) sont transféré(s), après avoir été séparé(s) de l'eau produite par la réaction d'hydrogénation, dans la zone IIIb où a lieu la réaction avec l'urée pour former les carbonates de propylène glycol cycliques à 5 ou 6 atomes selon la structure du propylène glycol ayant réagi.

Si l'on souhaite obtenir des carbonates d'éthylène glycol, il est possible de procéder en modifiant les conditions opérationnelles au niveau de la zone IIIa à une hydrogénolyse du glycérol dans des conditions plus sévères produisant de l'éthylène glycol qui est transformé par action de l'urée en un carbonate d'éthylène, cycle à 5 atomes. En pratique, les opérations d'hydrogénation du glycérol donnent le plus souvent un mélange de propylène glycol et d'éthylène glycol.

La zone IV comporte outre les lignes d'alimentation 15, 8 et 10, une ligne d'appoint 16 permettant notamment de fournir l'ammoniac nécessaire au démarrage de l'unité et à sa régulation en cas de variation de la production d'ammoniac au niveau de la zone III.

Le procédé est particulièrement bien adapté à la synthèse de nitriles et/ou amines gras. Les différentes réactions seront les suivantes :

Zone I : Huile végétale + 3R₁OH → R-COOR₁ + R'-COOR₁ + R"-COOR, + HOCH₂-CHOH-CH₂OH ,

R₁ étant CH₃ ou H , R, R' et R" identiques ou différents

Zone IIa : R-COOR, + H₂O → R-COOH + R₁OH avec R₁ = CH₃

Zone IIb : R-COOH + NH₃ → R-CN + 2 H₂O

Zone IIc : R-CN + 2 H₂ (NH₃) → R-CH₂-NH₂

Les mêmes réactions s'appliquant pour R' et R".

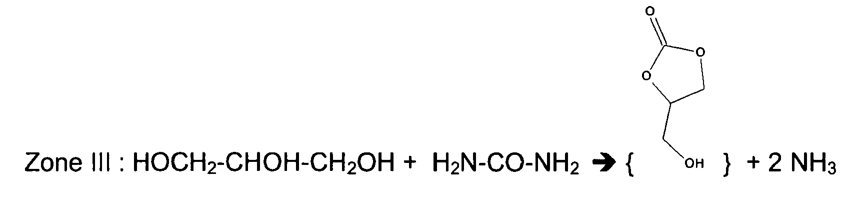

On pourra également utiliser le même procédé pour la production de nitriles/amines directement à partir d'ester ou de triglycéride. Pour cela les étapes IIa et/ou I seront éventuellement omises.

Les conditions de mise en oeuvre des différentes étapes des zones I, II, III et IV sont connues de l'homme de l'art.

On peut cependant préciser que :
- la méthanolyse de la zone I est généralement réalisée par exemple à une température comprise entre 60° et 100°C en présence d'un catalyseur du type méthylate de sodium et avec un excès de méthanol. Il est aussi possible d'utiliser une catalyse hétérogène à plus haute température - ou même une catalyse acide.
- l'hydrolyse de la zone I est généralement réalisée en présence d'un catalyseur acide.
- la réaction d'hydrolyse de l'étape a) de la zone II est réalisée par exemple à la température ambiante ou même à une température un peu plus élevée.
- l'ammoniation de l'étape b) dans la zone II conduisant au nitrile est réalisée à une température allant de 150° à 400°C, et de préférence à une température voisine de 300°C.
- l'hydrogénation de l'étape c) dans la zone II conduisant à une amine est réalisée à une température allant de 80° à 170°C, de préférence en présence d'ammoniac pour former l'amine primaire.
- la conversion du glycérol en carbonate de glycérol réalisée au niveau de la zone III est comme cela a été indiqué précédemment connue depuis plusieurs années. Pour la conduite du procédé, la solution retenue est la transestérification du glycérol par l'urée. Cette réaction est conduite en présence de catalyseurs connus en soi, par exemple ceux décrits dans les brevets US 6,495,703, oxyde de zinc, ou EP 0 955 298, sulfates métalliques, de zinc ou organométalliques.

Dans une variante, le procédé permet d'obtenir des carbonates de polyols autres que le glycérol, à savoir les diols comportant 3 atomes de carbone tels le 1,2-propane diol (ou 1,2-propylène glycol), le 1,3-propane diol et l'éthylène glycol (ou éthane diol), et notamment le 1,2-propylène glycol qui sont obtenus par hydrogénation du glycérol issu de la zone I. Cette hydrogénation est conduite selon des conditions bien connues de l'homme de l'art.

Plusieurs demandes de brevets et articles décrivent les conditions pour effectuer la transformation par hydrogénation du glycérol en propylène glycol, propane diol et éthylène glycol. On peut citer les brevets US 5,276,181, US 5,214,219, US 5,616,817, US 4,642,394 et les articles suivants : Daniel G. Lahr et col. J.Catal. 232 (2005), 386-394, Daniel G. Lahr et col. Ind. Eng. Chem. Res. (2003) 42,(22) ,5467-5472. Par ailleurs, des voies utilisant aussi des procédés de fermentation ont été décrites pour la transformation du glycérol en propane diol et propylène glycol, par exemple dans les articles suivants : D.C. Cameron et col. Biotechnology 4 (1986), 651, D.C. Cameron et col. Biotechnol. Prog. 14 (1998), 116.

Le brevet US 4,642,394 décrit un procédé de production d'un mélange de propane diols 1,2 et 1,3, à partir de glycérol par action d'un gaz de synthèse (H₂ + CO) dans un milieu solvant organique en présence d'un catalyseur soluble à base de tungstène et d'un métal du Groupe VIII.

Le brevet US 5,214,219 décrit un procédé de production d'un mélange de 1,2-propane diol et d'éthane diol à partir de glycérol par hydrogénation du glycérol en présence d'un catalyseur hétérogène zinc-cuivre dans un milieu aqueux ou solvant alcoolique.

Le brevet US 5,616,817 décrit un procédé de production de 1,2-propane diol à partir de glycérol en présence d'un catalyseur hétérogène à base de cobalt, de cuivre, de manganèse et de molybdène.

La transestérification du 1,2-propane diol par l'urée pour la synthèse du carbonate de propylène est décrite par Xinqiang Zhao et al dans un article intitulé « Synthesis of Propylene Carbonate from Urea and 1,2-Propylene Glycol over a Zinc Acetate Catalyst » Ind. Eng. Chem. Res., 43 (15), 4038-4042, 2004. Selon les mêmes auteurs dans un article paru dans Journal of Chemical Technology & Biotechnology en 2006 la même réaction peut être conduite avec un catalyseur oxyde double zinc-fer.

La transestérification de l'éthane diol avec l'urée a été décrite par Chiyoda et Mitsubishi Gas Chemical dans une publication de Chiyoda sur la synthèse de carbonate d'éthylène. (ACS 212th National Meeting, August 1996, Div Fuel Chem. Preprint, Vol 41, N°3, pp. 868-872). Des données expérimentales citées dans un brevet de Mistubishi Gas Chemical (US 5,489,702) montrent qu'à 145°C et sous 100 mm Hg, avec 2 h de temps de contact, et en utilisant un catalyseur oxyde de zinc, une conversion complète de l'urée est obtenue pour une sélectivité en carbonate d'éthylène supérieure à 97 % par rapport à l'urée comme par rapport à l'éthylène glycol.

## Revendications

1. Procédé conjugué de production de nitriles et/ou amines gras et de carbonates de polyol à partir d'une huile naturelle comportant les zones suivantes :
I) zone de méthanolyse ou d'hydrolyse d'une huile naturelle contenant un triglycéride d'au moins un acide gras introduite par la ligne (1), par le méthanol ou l'eau introduit par la ligne (2), produisant d'une part le méthanolate de l'acide gras ou l'acide gras répondant à la formule générale R-COOR₁, dans laquelle R est un radical alkyl saturé ou insaturé comportant de 7 à 21 atomes de carbone, R₁ est soit CH₃ dans le cas de la méthanolyse, soit H dans le cas de l'hydrolyse, et d'autre part du glycérol, qui sont respectivement adressés, après séparation au sein de la zone (Ib) aux zones (II) par la ligne (3) et zone (III) par la ligne (11).
II) zone de synthèse d'une amine grasse comportant les étapes suivantes a) transformation optionnelle tout d'abord du méthanolate de l'acide gras introduit par la ligne (3) en un acide gras par une hydrolyse de l'ester issu de l'étape I), l'eau étant introduite par la ligne (5) et le méthanol extrait par la ligne (6), suivie en b) d'une réaction d'ammoniation avec de l'ammoniac en excès introduit par la ligne (7) de l'acide ou du méthanolate de l'acide gras issu de la zone (I) introduit par la ligne (3) ou de l'acide issu de l'étape a) pour former un nitrile, l'excès d'ammoniac et l'eau produite étant extraits par la ligne (8) ou l'excès d'ammoniac et l'eau et le méthanol produits étant extraits par la ligne (14) et/ou (8), puis en c) d'une réduction par l'hydrogène introduit par la ligne (4) du composé résultant de l'étape IIb, éventuellement en présence d'ammoniac introduit par la ligne (17), pour obtenir l'amine correspondante extraite par la ligne (9), avec recyclage éventuel de l'ammoniac à la zone (IV) par la ligne (10),
III) zone de synthèse d'un carbonate de polyol par réaction de l'urée, soit directement sur le glycérol amené par la ligne (11), soit sur du propylène glycol ou de l'éthylène glycol obtenus suite à une réduction par hydrogénation du glycérol, la réaction du polyol avec l'urée produisant de l'ammoniac, le carbonate de polyol étant extrait par la ligne (13) et l'ammoniac formé adressé à la zone (IV) par la ligne (15),
IV) zone de récupération de l'ammoniac issue de la zone (III) par la ligne (15) ainsi que celle par la ligne (8) issue de la zone (IIb) où la réaction est réalisée avec un excès d'ammoniac et éventuellement celle par la ligne (10) issue de la zone (IIc), pour servir d'alimentation par la ligne (7) pour l'ammoniation de l'étape IIb et éventuellement par la ligne (17) pour l'hydrogénation de l'étape IIc de la zone (II).

2. Procédé selon la revendication 1 **caractérisé en ce que** la réaction dans la zone (I) est une méthanolyse pour obtenir un composé de formule R-COOCH₃ dans laquelle R est un radical alkyl saturé ou insaturé comportant de 7 à 21 atomes de carbone.

3. Procédé selon la revendication 1 **caractérisé en ce que** la réaction dans la zone (I) est une hydrolyse pour obtenir un composé de formule R-COOH dans laquelle R est un radical alkyl saturé ou insaturé comportant de 7 à 21 atomes de carbone.

4. Procédé selon l'une des revendications 1 à 3 **caractérisé en ce que** le glycérol issu de la zone (I) et adressé à la zone (III) est transformé en carbonate de glycérol par action de l'urée.

5. Procédé selon l'une des revendications 1 à 3 **caractérisé en ce que** le glycérol issu de la zone (I) et adressé à la zone (III) est réduit dans une zone (IIIa) par action de l'hydrogène en au moins un diol, propane diol et/ou éthane diol, ces derniers étant ensuite transformés en carbonates de propane diol et/ou éthane diol par action de l'urée.

## Claims

1. Conjugate process for producing fatty nitriles and/or amines and polyol carbonates from a natural oil, including the following zones:
I) zone for methanolysis or hydrolysis of a natural oil containing a triglyceride of at least one fatty acid introduced via line (1), with methanol or water introduced via line (2), producing, on the one hand, the methyl ester of the fatty acid or the fatty acid corresponding to the general formula R-COOR₁, in which R is a saturated or unsaturated alkyl radical containing from 7 to 21 carbon atoms, R₁ is either CH₃ in the case of methanolysis, or H in the case of hydrolysis, and, on the other hand, glycerol, which are, respectively, conveyed, after separation in zone (Ib), to the zones (II) via line (3) and zone (III) via line (11),
II) zone for synthesis of a fatty amine, including the following steps a) optional conversion firstly of the methyl ester of the fatty acid introduced via line (3) into a fatty acid via hydrolysis of the ester derived from step I), the water being introduced via line (5) and the methanol extracted via line (6), followed in b) by an ammoniation reaction with excess ammonia, introduced via line (7), of the acid or of the fatty acid methyl ester obtained from zone (I) introduced via line (3) or of the acid obtained from step a), to form a nitrile, the excess ammonia and the water produced being extracted via line (8) or the excess ammonia and the water and the methanol produced being extracted via line (14) and/or (8), and then in c) a reduction with the hydrogen introduced via line (4) of the compound resulting from step IIb, optionally in the presence of ammonia introduced via line (17), to obtain the corresponding amine, extracted via line (9), with optional recycling of the ammonia into zone (IV) via line (10),
III) zone for synthesis of a polyol carbonate by reaction of urea, either directly on the glycerol conveyed via line (11), or on propylene glycol or ethylene glycol obtained following a reduction by hydrogenation of glycerol, the reaction of the polyol with the urea producing ammonia, the polyol carbonate being extracted via line (13) and the ammonia formed conveyed to zone (IV) via line (15),
IV) zone for recovery of the ammonia obtained from zone (III) via line (15), and also that obtained via line (8) obtained from zone (IIb) in which the reaction is performed with an excess of ammonia, and optionally that via line (10) obtained from zone (IIc), to serve as feed via line (7) for the ammoniation of step IIb and optionally via line (17) for the hydrogenation of step IIc of zone (II).

2. Process according to Claim 1, **characterized in that** the reaction in zone (I) is a methanolysis to obtain a compound of formula
R-COOCH₃ in which R is a saturated or unsaturated alkyl radical containing from 7 to 21 carbon atoms.

3. Process according to Claim 1, **characterized in that** the reaction in zone (I) is a hydrolysis to obtain a compound of formula
R-COOH in which R is a saturated or unsaturated alkyl radical containing from 7 to 21 carbon atoms.

4. Process according to one of Claims 1 to 3, **characterized in that** the glycerol obtained from zone (I) and conveyed to zone (III) is converted into glyceryl carbonate via the action of urea.

5. Process according to one of Claims 1 to 3, **characterized in that** the glycerol obtained from zone (I) and conveyed to zone (III) is reduced in a zone (IIIa) via the action of hydrogen to at least one diol, propanediol and/or ethanediol, the latter then being converted into propanediol and/or ethanediol carbonates via the action of urea.

## Patentansprüche

1. Gekoppeltes Verfahren zur Produktion von Nitrilen und/oder Fettaminen und Polyolcarbonaten aus einem natürlichen Öl, das die folgenden Bereiche umfasst:
I) Bereich zur Methanolyse oder Hydrolyse eines natürlichen Öls, das ein Triglycerid mindestens einer Fettsäure enthält und über die Linie (1) eingeführt wird, mittels Methanol oder Wasser, die über die Linie (2) eingeführt werden, wodurch einerseits das Methanolat der Fettsäure oder die Fettsäure, die der allgemeinen Formel R-COOR₁ entspricht, worin R ein gesättigter oder ungesättigter Alkylrest ist, der 7 bis 21 Kohlenstoffatome umfasst, R₁ entweder CH₃, im Fall der Methanolyse, oder H ist, im Fall der Hydrolyse, und andererseits das Glycerol produziert wird, die jeweils nach Trennung innerhalb des Bereichs (Ib) in die Bereiche (II) über die Linie (3) und in den Bereich (III) über die Linie (11) geschickt werden.
II) Bereich zur Synthese eines Fettamins, die die folgenden Schritte umfasst: a) vorausgehende optionale Umwandlung des Methanolats der Fettsäure, das über die Linie (3) eingeführt wird, in eine Fettsäure, mittels einer Hydrolyse des Esters, der aus Schritt I) stammt, wobei das Wasser über die Linie (5) eingeführt wird und das Methanol über die Linie (6) extrahiert wird, gefolgt in b) von einer Ammonisierungsreaktion mit Überschussammoniak, der über die Linie (7) eingeführt wird, der Säure oder des Methanolats der Fettsäure, die (das) aus dem Bereich (I) stammt, die (das) über die Linie (3) eingeführt wird, oder der Säure, die aus Schritt a) stammt, um ein Nitril zu bilden, wobei der Ammoniaküberschuss und das produzierte Wasser über die Linie (8) extrahiert werden, oder der Ammoniaküberschuss und das Wasser und das produzierte Methanol über die Leitung (14) und/oder (8) extrahiert werden, dann in c) eine Reduktion mittels Wasserstoff, der über die Linie (4) eingeführt wird, der Verbindung, die aus Schritt IIb resultiert, gegebenenfalls in Gegenwart von Ammoniak, der über die Linie (17) eingeführt wird, um das entsprechende Amin zu erhalten, das über die Linie (9) extrahiert wird, mit möglicher Rückführung des Ammoniaks in den Bereich (IV) über die Linie (10),
III) Bereich zur Synthese eines Polyolcarbonats durch Umsetzung des Harnstoffs, oder direkt auf dem Glycerol, das über die Linie (11) geleitet wird, oder auf Propylenglykol oder Ethylenglykol, die nach einer Reduktion durch Hydrieren des Glycerols erhalten werden, wobei die Umsetzung des Polyols mit dem Harnstoff Ammoniak produziert, wobei das Polyolcarbonat über die Linie (13) extrahiert wird und der gebildete Ammoniak über die Linie (15) in den Bereich (IV) geschickt wird,
IV) Bereich zur Gewinnung des Ammoniaks, der aus dem Bereich (III) stammt, über die Linie (15) sowie desjenigen über die Linie (8) aus dem Bereich (IIb), wo die Umsetzung mit einem Ammoniaküberschuss ausgeführt wird und gegebenenfalls desjenigen über die Linie (10) aus dem Bereich (IIc), um der Versorgung über die Linie (7) für die Ammonisierung aus Schritt IIb und gegebenenfalls über die Linie (17) für die Hydrierung aus Schritt IIc des Bereichs (II) zu dienen.

2. Verfahren nach Anspruch 1, **dadurch gekennzeichnet, dass** die Reaktion im Bereich (I) eine Methanolyse ist, um eine Verbindung mit der Formel R-COOCH₃ zu erhalten, worin R ein gesättigter oder ungesättigter Alkylrest ist, der 7 bis 21 Kohlenstoffatome umfasst.

3. Verfahren nach Anspruch 1, **dadurch gekennzeichnet, dass** die Reaktion im Bereich (I) eine Hydrolyse ist, um eine Verbindung mit der Formel R-COOH zu erhalten, worin R ein gesättigter oder ungesättigter Alkylrest ist, der 7 bis 21 Kohlenstoffatome umfasst.

4. Verfahren nach einem der Ansprüche 1 bis 3, **dadurch gekennzeichnet, dass** das Glycerol, das aus dem Bereich (I) stammt und in den Bereich (III) geschickt wird, durch die Wirkung des Harnstoffs in Glycerolcarbonat umgewandelt wird.

5. Verfahren nach einem der Ansprüche 1 bis 3, **dadurch gekennzeichnet, dass** das Glycerol, das aus dem Bereich (I) stammt und in den Bereich (III) geschickt wird, in einem Bereich (IIIa) durch die Wirkung des Wasserstoffs in mindestens ein Diol, Propandiol und/oder Ethandiol reduziert wird, wobei diese letztgenannten anschließend durch die Wirkung des Harnstoffs in Propandiol- und/oder Ethandiolcarbonate umgewandelt werden.
